Europäisches Patentamt

European Patent Office

Office européen des brevets

(19)

(11) Veröffentlichungsnummer: **0 187 361**
**A2**

# (12) EUROPÄISCHE PATENTANMELDUNG

(21) Anmeldenummer: 85116422.8

(22) Anmeldetag: 21.12.85

(51) Int. Cl.⁴: **A 61 K 9/22**

(30) Priorität: 05.01.85 DE 3500268

(43) Veröffentlichungstag der Anmeldung:
16.07.86 Patentblatt 86/29

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Grau, Ulrich, Dr.
Am Forsthaus 1
D-6238 Hofheim am Taunus(DE)

(54) Präparate mit verzögerter Wirkung, Verfahren zu deren Herstellung sowie entprechende Mittel zur Human- bzw. veterinärmedizinischen Anwendung.

(57) Die Erfindung betrifft protrahiert wirkende sterile wäßrige Zubereitungen eines Peptids oder Proteins, gekennzeichnet durch den Gehalt an einem physiologisch verträglichen Geliermittel in einer für eine Depotwirkung aus reichenden Menge, Verfahrzen zu deren Herstellung und deren Verwendung.

EP 0 187 361 A2

Präparate mit verzögerter Wirkung, Verfahren zu deren Herstellung sowie entsprechende Mittel zur Human- bzw. veterinärmedizinischen Anwendung

Bei der Anwendung von Arzneimitteln am Menschen oder Tier ist es manchmal erwünscht, dem eigentlichen Pharmakon durch geeignete Maßnahmen eine verlängerte Wirkung in vivo zu verleihen. Dieses Problem ist insbesondere bei Proteinen oder Peptiden von großer Bedeutung, da diese normalerweise relativ kurze Halbwertzeiten besitzen, da sie in vivo z.T. schnell metabolisiert werden. Ein typisches Beispiel für diese Substanzklasse ist das Insulin, bei dessen parenteraler Applikation ohne Depotprinzip 3 bis 4 Injektionen am Tag nötig sind, um eine Stoffwechselentgleisung des Diabetikers zu verhindern. Durch Zusatz eines Depotträgers kann die Wirkung des Insulins dagegen erheblich verlängert werden.

Als Depotträger kommen beispielsweise beim Insulin stoffspezifische Verzögerungsprinzipien in Frage, die die Schwerlöslichkeit des Insulins bei physiologischem pH-Wert bedingen. Weitere geläufige und allgemein anwendbare Depotformen für Proteine oder Peptide sind sogenannte drug delivery systems. Solche Systeme bestehen aus einer Abgabevorrichtung, i.a. einem Hohlkörper oder Polymernetzwerk, in den das Pharmakon eingebettet ist und durch aktiven oder passiven Transport in den Körper gebracht wird.

Diese Systeme sind üblicherweise fest und werden für Proteine oder Peptide zum Beispiel unter die Haut gespritzt oder implantiert. Dazu zählen die Methoden der Mikroverkapselung (F. Lim, Biomedical Application of Microencapsulation, CRC Press, Boca Raton (1984)), Polymerimplantate (R.Langer und J.Folkmann, Nature 263, 797-800 (1976),

M.F.A. Goosen et al, Diabetes 32, 418-481 (1983)),
erodierbare Polymermatrizes (R.W. Baker et al, Pharmaceutical Technology, 26-30 (1984)), Hydrogele (EP -A2-
0092918), osmotische Dispenser (US-Patent 3995632), oder
auch andere Systeme, die bislang bevorzugt für niedermolekulare Pharmaka Anwendung fanden (Selecta 21, 1966-81
(1983), N.L. Henderson, in Topics in Chemistry and Drug
Design, R. C. Allen, ed, (1983)).

Ziel der "drug delivery systems" ist es, das Pharmakon
über lange Zeit mit einer Kinetik nahe nullter Ordnung
abzugeben. Häufig wiederkehrende Probleme sind aber die
Reproduzierbarkeit der Systeme, z. B. der Porengröße, und
damit die Reproduzierbarkeit der Abgabecharakteristik,
eine imperfekte Kinetik, z.B. eine Kinetik 1. Ordnung mit
deutlichem Peak zu Anfang und ein langsames Abnehmen
gegen Ende der Abgabezeit, sowie die Erhaltung der Aktivität und Langzeitstabilität des Wirkstoffs. Schließlich
sind einige der o.g. Methoden auf Proteine und Peptide
nur mit Aktivitätsverlust anwendbar.

Aus der EP-A3-0019403 ist weiterhin bekannt, Peptiden wie
Insulin und Enkephalin eine verzögernde Wirkung dadurch
zu verleihen, daß man sie kovalent an einen Hydroxyethyl-
Eisen(III)-Komplex bindet.

Aus der DE-A-27 58 578 und dem kanadischen Patent 1107647
sind Sekretinzubereitungen bekannt, die zur Stabilisierung Glycin und geringe Mengen Haemaccel$^R$, ein mit Diisocyanaten vernetztes Gelatinepartialhydrolysat, enthalten.
Außerdem wird ein Depotträger aus Polyphloretinphosphat u.
Haemaccel beschrieben. Ferner sind aus dem US-Patent
4 302 448 Sekretinpräparate mit protrahierter Wirkung bekannt, die einen phenolischen Depotkörper und zusätzlich
bis zu 10% eines Gelatinederivats enthalten. Kraegen et
al. berichten in Brit. Med. J. 1975 III 464-466 von einem stabilisierenden Effekt eines Zusatzes von bis zu 3,5 % Haemaccel zu sehr verdünnten Insulinlösungen (0,04 I.E./ml), wodurch sich unter anderem die Adsorption des Insulins am Vorratsgefäß und Schlauchsystem in Infusionsystemen verhindern läßt.

In der EP-A-21234 werden wäßrige Lösungen von LHRH-Analogen beschrieben, die 0,005 % Gelatine enthalten.

Wäßrige Insulinzubereitungen mit verzögerter Wirkung, gekennzeichnet durch einen Gehalt eines physiologisch verträglichen Geliermittels, sind bereits vorgeschlagen worden (deutsche Patentanmeldung P 3 421 613.8).

Aufgabe der vorliegenden Erfindung ist es nun, ein einfaches und reproduzierbares Depotkonzept mit mittellanger, typischerweise über Stunden bis wenige Tage andauernder Wirkung zu formulieren, das generell für höhermolekulare Arzneistoffe, bevorzugt aber für die z.T. recht empfindlichen Proteine oder Peptide bzw. deren Analoge, insbesondere bei deren parenteralen, vorzugsweise subkutaner oder intramuskulärer Applikation, anwendbar ist.

Diese Aufgabe wurde erfindungsgemäß dadurch gelöst, daß man wäßrigen Arzneimittelzubereitungen ein physiologisch verträgliches Geliermittel als Depotprinzip zusetzt.

Die Erfindung betrifft daher

protrahiert wirkende sterile wäßrige Zubereitungen eines Peptids oder Proteins, die dadurch gekennzeichnet sind, daß sie als alleiniges Depotprinzip ein physiologisch verträgliches Geliermittel in einer für eine Depotwirkung ausreichenden Menge enthalten, wobei Insulinzubereitungen ausgenommen sind.

Als Geliermittel (oft auch Verdickungsmittel genannt) kommen physiologisch verträgliche Polymere in Frage, wie Kollagen und Folgeprodukte, Dextrane, andere Polysaccharide, wie etwa Laevane, Gelatine, Oxypolygelatine (Gelifundol[R]), modifizierte flüssige Gelatine (Physiogel[R]), Gelatinepartialhydrolysate, beispielsweise mit Diisocyanaten vernetzte Gelatinepartialhydrolysate (Polygeline, Haemaccel[R]), Hydroxyethylstärken oder Polyvinylpyrrolidon. Ein Teil der genannten Substanzen findet auch in kolloidalen Plasmaersatzmitteln Verwendung.

Die erfindungsgemäßen Zubereitungen können verflüssigt oder in Form von Hydrogelen vorliegen. Überraschenderweise wurde gefunden, daß insbesondere auch solche Gel-Zubereitungen, die bei Körpertemperatur flüssig vorliegen und deshalb leicht injizierbar sind, den Peptiden und Proteinen eine verzögerte und ggf. verstärkte Wirkung verleihen. Sie enthalten vorteilhaft mehr als 1 %, vorzugsweise mehr als 2 %, insbesondere zwischen 5 und 25 % Geliermittel. Doch können auch beim Zusatz geringerer Mengen Geliermittel schon formbeständige Hydrogele entstehen; beispielsweise genügt hierzu der Zusatz von etwa 0,2 % Agar oder 0,6 % Gelatine. Nach oben hin kann der Gehalt an Geliermittel, abhängig von dessen Art, 30 % und mehr betragen (%-Angaben in Gewichtsprozent). Insbesondere bei den kleineren Peptiden sind hohe Gelanteile zur Erzielung eines signifikanten Depoteffekts nötig.

Bevorzugte Geliermittel sind mit Diisocyanaten vernetzte Gelatinepartialhydrolysate (vgl. z.B. DBP 1 118 792 und 1 155 134), wie Polygeline. Man stellt sie her

1) durch Umsetzung von Kollagen-Abbauprodukten von einem Molekulargewicht von 2000 - 20 000 mit einer geringeren Menge Diisocyanat, als der Anzahl der im abgebauten Kollagen vorhandenen Amino- und Guanidino-Gruppen entspricht oder

2) durch weiteren Abbau des nach 1) erhaltenen Vernetzungsproduktes in wäßriger Lösung bis zu einem
Molekulargewicht von 10 000 bis 100 000.

Man setzt die bevorzugten vernetzten Gelatinepartialhydrolysate gewöhnlich in Gewichtsmengen von mehr als 1 %,
vorzugsweise mehr als 2 % zu. Bei kleineren Peptiden,
wie z.B. Sekretin, ist es vorteilhaft, wenn der Anteil
dieses Geliermittels mehr als 5 Gewichts-%, vorzugsweise
mehr als 10 % beträgt.

Die erfindungsgemäßen Zubereitungen enthalten das Geliermittel und das Pharmakon üblicherweise in einem wäßrigen
Medium, das zusätzlich eine Puffersubstanz, ein Konservierungsmittel, ein Isotoniemittel sowie, falls erforderlich, zusätzliche Stabilisatoren oder weitere in anderer
Weise übliche und bekannte Zusätze enthalten kann. Das
Medium kann dabei einen vom Neutralpunkt abweichenden
pH-Wert aufweisen , jedoch ist ein pH-Wert von 3 - 10,
insbesondere von 6 - 8 bevorzugt, weil Proteine und Peptide häufig dort am stabilsten und pH-neutrale Lösungen
physiologisch am besten verträglich sind.

Anders als bei konventionellen Depotformen handelt es
sich hier also im allgemeinen um klar gelöste Zubereitungen, vor deren Injektion kein Homogenisieren nötig ist.
Bei Suspensionen kommen bekanntlich Dosierungsfehler
wegen ungenügender Homogenisierung vor. Gelartige Zubereitungen lassen sich prinzipiell aber auch mit amorphem
oder kristallinem Wirkstoff herstellen. Damit ist es
möglich, schwerlösliche Formen des Wirkstoffs, z.B.
Kristalle oder amorphe Niederschläge in Form eines Gels
zuzubereiten. So können verschiedene Depotformen kombiniert werden und ultralang wirkende "trübe Gele" hergestellt werden.

Bei genügend hohem Anteil an Geliermittel ist allen Zubereitungen gemeinsam, daß sie unter Lagerungsbedingungen fest als Hydrogel vorliegen. Da die Moleküle quasi "eingefroren" und nur langsam an die Gefäßwand oder die Grenzfläche Flüssigkeit/Luft diffundieren können, und da Bewegungen und Turbulenzen innerhalb der Gele während der Handhabung erheblich reduziert sind, weisen diese eine besondere Lagerstabilität auf. Vor der Anwendung werden die Gele, wie auch bei konventionellen Insulinen üblich, auf Raum- bis Körpertemperatur gebracht, wobei sie sich verflüssigen, aber trotzdem noch eine gegenüber konventionellen Lösungen der Wirkstoffe erhöhte Viskosität aufweisen.

Die Gelbildung unter Lagerungsbedingungen kann auch hinsichtlich der Homogenität der Lösung bzw. Suspension vorteilhaft sein. Es ist z.B. bei sedimentierten Kristallsuspensionen bei langer Lagerung durchaus denkbar, daß relativ stabile Kristallassoziate sich bilden, die dann weniger leicht homogen aufschüttelbar sind und dadurch zu Dosierungsfehlern führen können. Liegt dagegen die Kristallsuspension homogen in einem Gel "eingefroren" vor, so sind solche Effekte auszuschließen.

Als Pharmaka kommen insbesondere höhermolekulare Stoffe, bevorzugt Proteine und Peptide für die Human- und Veterinärmedizin in Frage, die in einem wäßrigen Medium aufgrund ihrer überwiegend hydrophilen Natur besonders stabil sind. Dazu zählen beispielsweise Hormone bzw. Releasing-Hormone und deren Analoge wie Sekretin, Gastrin, Oxytocin, Differenzierungsfaktoren, Wachstumshormon, GHRH, LHRH, TRH, Somatostatin, Glucagon, Neurotensin, Calcitonin, Enkephaline, ACTH, Bradykinin, Cholecystokinin, Erythropoietin, Thymuspeptide und Relaxin; Proteine mit anderen Funktionen wie Immunglobuline verschiedener Klassen und Spezies, Albumin, Blutgerinnungsfaktoren (z.B. Hirudin, Plasminogen-Aktivator, Antithrombin III), aneurexogene
Peptide, Interleukine und Interferone; sowie Enzyme wie
Neuraminidase, Glucosidase, Glycokinase, Myoglobin, Lysozym
und Uricase.

Als physiologisch unbedenkliches und mit den Wirkstoffen
verträgliches Trägermedium eignet sich eine sterile wäßrige Lösung, die zu Blut in der üblichen Weise, z.B. durch
Glycerin, Kochsalz, Glucose, isotonisch gemacht wird und
daneben noch eines der gebräuchlichen Konservierungsmittel
z.B. Phenol, m-Kresol oder p-Hydroxybenzoesäureester in
einer für diesen Zweck üblichen Menge, enthält. Das Trägermedium kann zusätzlich eine Puffersubstanz, z. B.
Natriumacetat, Natriumcitrat, Natriumphosphat, Tris-
(hydroxymethyl)-aminomethan, enthalten. Zur Einstellung
des pH werden verdünnte Säuren (typischerweise HCl) bzw.
Laugen (typischerweise NaOH) verwendet.

Die physikalische Stabilität der erfindungsgemäßen Zubereitungen kann ggf. weiter erhöht werden durch Zusatz von
oberflächenaktiven Substanzen, wie sie z.B. aus der
EP-A-18 609, der DE-A-32 40 177, der DE-A-29 17 535 oder
der WO-A-83/00288 bekannt sind.

Die Erfindung betrifft weiterhin ein Verfahren zur Herstellung einer protrahiert wirkenden sterilen wäßrigen
Peptid- oder Protein-Zubereitung, das dadurch gekennzeichnet ist, daß man einer wäßrigen Zubereitung eines
Peptids oder Proteins ein physiologisch verträgliches
Geliermittel und ggf. ein geeignetes Isotoniemittel,
einen geeigneten Puffer, ein geeignetes Konservierungsmittel und/oder weitere Hilfsstoffe zusetzt und die
Lösung beispielsweise durch Sterilfiltration sterilisiert.

Ebenso ist es möglich, sterile wäßrige Peptid- oder
Proteinzubereitungen mit sterilen Zubereitungen des Geliermittels und ggf. weiterer Hilfsstoffe zu vereinigen.

Die Erfindung betrifft ferner die Verwendung der erfindungsgemäßen Zubereitungen als Heilmittel (für Sekretinzubereitungen z.B. zur Behandlung von Pankreatitis oder Mucoviszidose), die Verwendung eines physiologisch verträglichen Geliermittels als Depothilfsmittel sowie die Verwendung von Geliermitteln als stabilisierendes Mittel bei der Handhabung und Lagerung insbesondere von Hydrogelen.

Die erfindungsgemäßen Wirkstoffzubereitungen können parenteral, insbesondere subkutan oder intramuskulär appliziert werden. Der Depoteffekt zeigt sich am ausgeprägtesten bei der subkutanen Applikationsweise, ist aber noch deutlich bei intramuskulärer Injektion. Es ist dabei überraschend, daß neben einer Wirkungsverlängerung oft auch eine Verstärkung der Wirkung zu beobachten ist. Intravasal appliziert wirken die erfindungsgemäßen klar gelösten Zubereitungen gewöhnlich rasch. Sie können daher auch in automatischen Dosiereinheiten, wie z.B. Pumpen, Verwendung finden. Dabei ist von Vorteil, daß die physikalische Stabilität in vielen Fällen durch Zusatz von Geliermitteln erhöht wird.

Die folgenden Beispiele sollen den Erfindungsgedanken weiter erläutern, ohne daß darin eine Einschränkung auf diese Beispiele beinhaltet wäre.

Beispiel 1

Herstellung einer Sekretinzubereitung mit 20 Gew.-%
Polygeline

200 g Polygeline und 2,5 g m-Kresol wurden bei ca. 30-40°C
in 800 ml Wasser p.i. gelöst und mit 1N HCl wird pH 3
eingestellt. Die Lösung wurde auf 20°C abgekühlt und
100 000 KE Sekretin · HCl unter Rühren zugegeben. Die Lösung wurde durch ein Sterilfilter sterilfiltriert und
in Mehrfachentnahmeflaschen abgefüllt.

Beispiel 2

Herstellung einer Lösung eines LHRH-Analogen mit
20 Gew.-% Dextran 60

20 g Dextran 60 und 0,9 g Benzylalkohol wurden in 80 ml
Wasser p.i. gelöst, auf pH 7 eingestellt und 25 mg des
LHRH-Analogen Buserelin zugegeben. Die Lösung wurde
sterilfiltriert und in Mehrfachentnahmeflaschen abgefüllt.

Beispiel 3

a) Herstellung einer Sekretinzubereitung mit 200g
   Polygeline/l

Analog der in Beispiel 1 angegebenen Verfahrensweise wurde
eine sterile wäßrige Zubereitung mit folgender Zusammensetzung hergestellt:

| | |
|---|---|
| Sekretin · HCl | 100 000 KE |
| Polygeline, lyophylisiert | |
| (Behringwerke AG, Marburg) | 200 g |
| $NaH_2PO_4$ · $2H_2O$ | 2,1 g |
| Glycerin | 10 g |
| m-Kresol | 2 g |

aqua pro injectione ad 1000 ml, pH=7,2

b) Pharmakologische Vergleichsversuche

   i)   Methode Beagle-Hunden, denen zuvor eine chronische Pankreasfistel angelegt worden war, und urethanbetäubte Ratten erhielten die nach (a) hergestellte Lösung subkutan appliziert. Als Vergleich wurde eine entsprechende Sekretinzubereitung ohne Polygeline appliziert. Das abfließende Pankreassekret wurde in zeitlich gestaffelten Perioden gesammelt und die Serum-Sekretinspiegel radioimmunologisch gemessen.

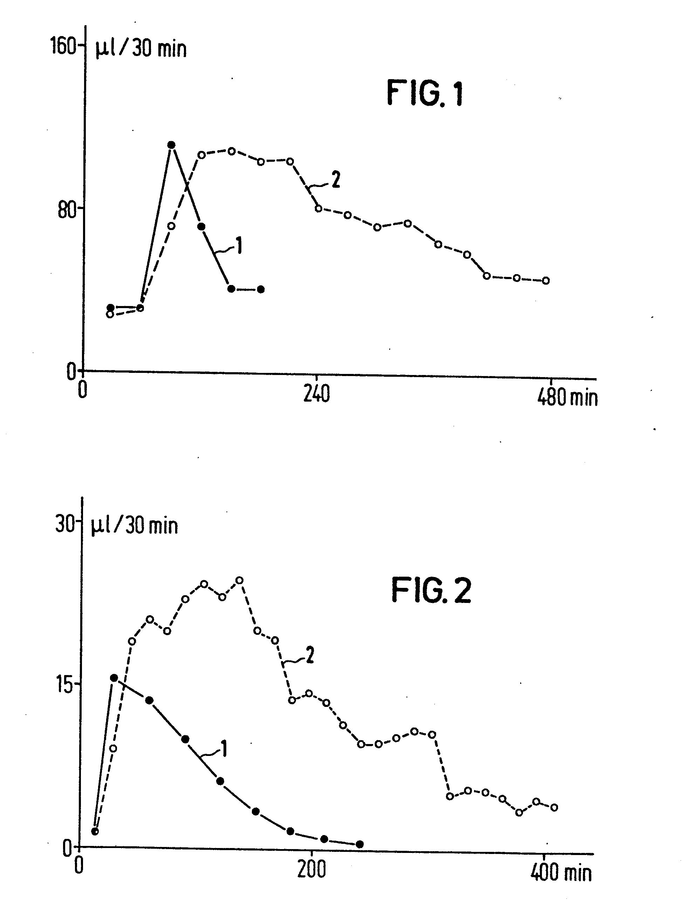

   ii)   Ergebnisse Die subkutane Applikation von 10 KE/kg Sekretin (®Sekretolin)in der Zubereitung ohne Polygeline bewirkte an der Ratte eine 2,5-stündige Pankreassekretion, dieser Effekt konnte durch Applikation des Peptides in der nach (a) hergestellten Zubereitung mit Polygeline auf 12 Stunden verlängert werden (Fig. 1). Analog konnte am wachen Hund die Wirkdauer von Sekretin (100 KE/Tier) von 3 auf über 10 Stunden verlängert werden (Fig. 2). Die über die Wirkdauer integrierte Präparatwirkung wurde gegenüber der Gabe des Vergleichs ohne Polygeline versechsfacht. Die Sekretin-Serum-Kinetik korrelierte mit den im Bioassay erhobenen Befunden.

   iii)   Schlußfolgerung Mit der vorliegenden Präparation kann bei zweimal täglicher ssc. Injektion eine 24-stündige Sekretinwirkung aufrechterhalten werden.

   iv)   Erläuterung zu den Abbildungen In Fig. 1 ist der zeitliche Verlauf der Pankreassekretion an der urethanbetäubten Ratte in µl/30min wiedergegeben. Kurve 1 zeigt den zeitlichen Sekretionsverlauf nach s.c. Gabe von 10 KE/kg der Vergleichszubereitung, während aus Kurve 2 der Verlauf nach s.c. Gabe von 10 KE/kg der Zubereitung gemäß (a) ersichtlich ist.

In Fig. 2 sind in gleicher Weise die Verhältnisse am wachen Hund nach s.c. Gabe von jeweils 100 KE/ Tier der Vergleichszubereitung bzw. der Zubereitung gemäß (a) wiedergegeben.

Beispiel 4

Herstellung eines Sekretinpräparats zur Behandlung der Mucoviszidose

Es wurde eine Depotträgerlösung folgender Zusammensetzung hergestellt:

| | |
|---|---|
| Polygeline (Behringwerke AG) | 200,0 g |
| Benzylalkohol | 10,0 g |
| $NaH_2PO_4 \cdot 2 H_2O$ | 2,1 g |
| aqua dest. p.i. ad | 1,0 l, pH = 6,4 |

in üblicher Weise sterilfiltriert und in sterile Mehrfachentnahmeflaschen abgefüllt.

Getrennt wurde eine sterile Lösung von

| | |
|---|---|
| Sekretin | 1 Million KE |
| Glycin | 20,0 g |
| Gelatinederivat | 2,0 g |
| aqua dest. p.i. ad | 1,0 l |

in 1 ml Aliquot in sterilen Glasampullen lyophilisiert und die Ampullen zugeschmolzen.

Vor der Applikation wird der Inhalt einer Sekretin-Ampulle in 1 ml Depotträger aufgenommen und anschließend s.c. oder i.m. injiziert.

Beispiel 5

Herstellung eines α-Interferonpräparats in einem
Polygeline-Träger

Es wurde eine Lösung folgender Zusammensetzung hergestellt:

| | |
|---|---|
| Human-α-Hybrid-Interferon | $10^9$ U |
| Polygeline (Behringwerke AG) | 15,00 g |
| Benzylalkohol | 1,00 g |
| Kochsalz | 0,60 g |
| Polyethylen-Polypropylenglykol 18oo | 0,0010 g |
| aqua dest. p.i.   ad | 100,00 ml, pH=7,2 |

Die Lösung wurde über ein vorher mit einer 0,1 % Poly-
ethylen-Polypropylenglykol 18oo-Lösung und dann mit einer
Placebolösung obiger Zusammensetzung (ohne Interferon)
voräquilibriertes Sterilfilter sterilfiltriert und in
sterile Mehrfachentnahmeflaschen abgefüllt.

Patentansprüche

1. Protrahiert wirkende sterile wäßrige Zubereitung eines Peptids oder Proteins, dadurch gekennzeichnet, daß sie als alleiniges Depotprinzip ein physiologisch verträgliches Geliermittel in einer für eine Depotwirkung ausreichenden Menge enthält, wobei Insulinzubereitungen ausgenommen sind.

2. Mittel gemäß Anspruch 1, dadurch gekennzeichnet, daß es als Hydrogel vorliegt.

3. Mittel gemäß Anspruch 1, dadurch gekennzeichnet, daß es in verflüssigter Form vorliegt.

4. Mittel gemäß einem der Ansprüche 1 bis 3, dadurch gekennzeichnet, daß es als Geliermittel Gelatine, Dextran, Laevan, andere Polysaccharide, Gelatinepartialhydrolysate, die vernetzt sein können, Oxypolygelatine, Hydroxyethylstärke oder Polyvinylpyrrolidon enthält.

5. Mittel gemäß Anspruch 4, dadurch gekennzeichnet, daß es ein mit Diisocyanaten vernetztes Gelatinepartialhydrolysat enthält.

6. Mittel gemäß Anspruch 5, dadurch·gekennzeichnet, daß es mehr als 1 Gewichts-%, vorzugsweise mehr als 2 Gewichts-% Geliermittel enthält.

7. Mittel gemäß einem der Ansprüche 1 bis 6, dadurch gekennzeichnet, daß dieses ein geeignetes Isotoniemittel, einen geeigneten Puffer und/oder ein geeignetes Konservierungsmittel enthält.

8. Mittel gemäß einem der Ansprüche 1 bis 7, dadurch gekennzeichnet, daß es als Wirkstoff Sekretin enthält.

9. Mittel gemäß einem der Ansprüche 1 bis 8 zur Anwendung als Heilmittel.

10. Verfahren zur Herstellung eines Mittels gemäß einem der Ansprüche 1 bis 9, dadurch gekennzeichnet, daß man einer wäßrigen Zubereitung eines Peptids oder Proteins ein physiologisch verträgliches Geliermittel und gegebenenfalls ein geeignetes Isotoniemittel, einen geeigneten Puffer und/oder ein geeignetes Konservierungsmittel zusetzt und die Zubereitung sterilisiert oder ein Peptid oder Protein zusetzt zu einer wäßrigen Zubereitung eines physiologisch verträglichen Geliermittels, welche gegebenenfalls ein geeignetes Isotoniemittel, einen geeigneten Puffer und/oder ein geeignetes Konservierungsmittel enthält, und die Zubereitung sterilisiert.

11. Verwendung eines physiologisch verträglichen Geliermittels als Depothilfsmittel in wäßrigen Zubereitungen von Peptiden oder Proteinen, Insulinzubereitungen ausgenommen.

12. Verwendung eines Geliermittels als stabilisierendes Mittel bei der Lagerung von Hydrogelen gemäß Anspruch 2.

PATENTANSPRÜCHE ÖSTERREICH:

1. Verfahren zur Herstellung einer protrahiert wirkenden sterilen wäßrigen Zubereitung eines Peptids oder Proteins, die als alleiniges Depotprinzip ein physiologisch verträgliches Geliermittel in einer für eine Depotwirkung ausreichenden Menge enthält, wobei Insulinzubereitungen ausgenommen sind, dadurch gekennzeichnet, daß man einer wäßrigen Zubereitung eines Peptids oder Proteins ein physiologisch verträgliches Geliermittel und gegebenenfalls ein geeignetes Isotoniemittel, einen geeigneten Puffer und/oder ein geeignetes Konservierungsmittel zusetzt und die Zubereitung sterilisiert und ein Peptid oder Protein zusetzt zu einer wäßrigen Zubereitung eines physiologisch verträglichen Geliermittels, welche gegebenenfalls ein geeignetes Isotoniemittel, einen geeigneten Puffer und/oder ein geeignetes Konservierungsmittel enthält, und die Zubereitung sterilisiert.

2. Verfahren gemäß Anspruch 1, dadurch gekennzeichnet, daß man ein Mittel herstellt, welches als Hydrogel vorliegt.

3. Verfahren gemäß Anspruch 1, dadurch gekennzeichnet, daß man ein Mittel herstellt, welches ein verflüssigter Form vorliegt.

4. Verfahren gemäß einem der Ansprüche 1 bis 3, dadurch gekennzeichnet, daß man ein Mittel herstellt, welches als Geliermittel Gelatine, Dextran, Laevan, andere Polysaccharide, Gelatinepartialhydrolysate, die vernetzt sein können, Oxypolygelatine, Hydroxyethylstärke oder Polyvinylpyrrolidon enthält.

5. Verfahren gemäß Anspruch 4, dadurch gekennzeichnet, daß man ein Mittel herstellt, welches ein mit Diisocyanaten vernetztes Gelatinepartialhydrolysat enthält.

6. Verfahren gemäß Anspruch 5, dadurch gekennzeichnet, daß man ein Mittel herstellt, welchen mehr als 1 Gewichts-%, vorzugsweise mehr als 2 Gewichts-% Geliermittel enthält.

7. Verfahren gemäß einem der Ansprüche 1 bis 6, dadurch gekennzeichnet, daß man ein Mittel herstellt, welches ein geeignetes Isotoniemittel, einen geeigneten Puffer und/oder ein geeignetes Konservierungsmittel enthält.

8. Verfahren gemäß einem der Ansprüche 1 bis 7, dadurch gekennzeichnet, daß man ein Mittel herstellt, welches als Wirkstoff Sekretin enthält.

9. Verfahren gemäß einem der ansprüche 1 bis 8 zur Herstellung eines Mittels zur Anwendung als Heilmittel.

10. Verwendung eines physiologisch verträglichen Geliermittels als Depothilfsmittel in wäßrigen Zubereitungen von Peptiden oder Proteinen, Insulinzubereitungen ausgenommen.

11. Verwendung eines Geliermittels als stabilisierendes Mittel bei der Lagerung von Hydrogelen gemäß Anspruch 2.

0187361

FIG.1

FIG.2